(19) Europäisches Patentamt
European Patent Office
Office européen des brevets

(11) Veröffentlichungsnummer: **0 000 934**
**B1**

# (12) EUROPÄISCHE PATENTSCHRIFT

(21) Anmeldenummer: **78100703.4**

(22) Anmeldetag: **18.08.78**

(51) Int. Cl.³: **C 07 C 125/06,**
**A 01 N 47/18, //**
**C 07 C 39/17,**
**C 07 C 39/24**

## (54) Indan-5-yl-N-alkyl-carbaminsäureester, Verfahren zu ihrer Herstellung sowie ihre Verwendung als Pflanzenschutzmittel

(30) Priorität: **31.08.77 DE 2739193**

(43) Veröffentlichungstag der Anmeldung:
**07.03.79 Patentblatt 79/05**

(45) Bekanntmachung des Hinweises auf die Patenterteilung:
**23.07.80 Patentblatt 80/15**

(84) Benannte Vertragsstaaten:
**BE CH DE FR GB NL**

(56) Entgegenhaltungen:
**DE - A - 2 603 835**

(73) Patentinhaber: **Bayer AG**
**Zentralbereich Patente, Marken und Lizenzen**
**D - 5090 Leverkusen 1, Bayerwerk (DE)**

(72) Erfinder: **Grotkopp, Detlef, Dr.**
**Cecilienallee 52**
**D - 4000 Düsseldorf (DE)**
**Wedemeyer, Karlfried, Dr.**
**Bilharzstrasse 7**
**D - 5000 Köln 80 (DE)**
**Brandes, Wilhelm, Dr.**
**Eichendorffstrasse 3**
**D - 5653 Leichlingen (DE)**
**Scheinpflug, Hans, Dr.**
**Am Thelenhof 15**
**D - 5090 Leverkusen 1 (DE)**
**Roessler, Peter, Dr.**
**Elsterstrasse 15**
**D - 5060 Berg. Gladbach (DE)**

**EP 0 000 934 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Indan-5-yl-N-alkyl-carbaminsäureester, Verfahren zu ihrer Herstellung sowie ihre Verwendung als Pflanzenschutzmittel

Die vorliegende Erfindung betrifft neue Indan-5-yl-N-alkyl-carbaminsäureester, mehrere Verfahren zu ihrer Herstellung sowie ihre Verwendung als Pflanzenschutzmittel.

Es ist bereits bekannt geworden, daß der N-Methyl-carbaminsäureester des 5-Hydroxy-indans insektizid wirksam ist (vgl. die US-Patentschriften 2 870 057 und 3 084 096). Weiterhin ist bereits bekannt geworden, daß das N-Trichlormethylthiotetrahydrophthalimid starke fungizide Eigenschaften aufweist (vgl. Science, Bd. *115,* S. 84 (1952)). Dieser in der Praxis wichtige Wirkstoff hat jedoch nur eine protektive Wirksamkeit.

Wirkstoffe, die die Metamorphose von Arthropoden hemmen, sind erst seit jüngerer Zeit im Pflanzenschutz von Interesse Zu nennen ist hier z.B. das 2,2-Dimethyl-6-methoxybenzopyran (Chem. Eng. News *54,* 19—20 (1976)).

Es wurden nun als neue Stoffe die Indan-5-yl-N-alkyl-carbaminsäureester der Formel

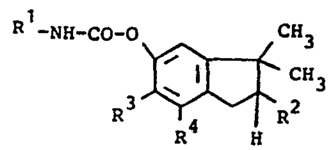

(I)

in welcher
$R^1$ für Alkyl steht,
$R^2$ für Wasserstoff oder Alkyl steht, und
$R^3$ und $R^4$ für Chlor oder Alkyl stehen,
gefunden.

Die Verbindungen weisen starke fungizide und die Entwicklung von Arthropoden hemmende Eigenschaften auf und sind daher als Pflanzenschutzmittel verwendbar.

Weiterhin wurde gefunden, daß man die Indan-5-yl-N-alkyl-carbaminsäureester der Formel (I) erhält, wenn man

a) 5-Hydroxy-indane der Formel

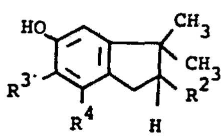

(II)

in welcher
$R^2$, $R^3$ und $R^4$ die oben angegebene Bedeutung besitzen, mit einem Alkylisocyanat umsetzt,
oder

b) 5-Hydroxy-indane der Formel (II) in einer ersten Stufe mit einem Überschuß an Phosgen in den entsprechenden Chlorkohlensäureester überführt und diesen mit Alkylamin umsetzt,
oder

c) 5-Hydroxy-indane der Formel (II) in einer ersten Stufe mit der äquivalenten Menge Phosgen zu dem entsprechenden Bis-(indanyl)-carbonat umsetzt und dieses in einer zweiten Stufe mit Alkylamin aufspaltet.

Die erfindungsgemäßen Indan-5-yl-N-alkyl-carbamate weisen eine überraschend hohe fungizide Wirkung auf und sind dem vorbekannten N-Trichlormethylthio-tetrahydropthalimid auch bei niedrigen Aufwandmengen überlegen. Weiterhin können sie nicht nur als protektive, sondern auch als kurative Mittel Verwendung finden. Die erfindungsgemäßen Stoffe stellen somit eine Bereicherung der Technik dar. Für die Verwendung als Pflanzenschutzmittel ist von Interesse, daß die Wirkstoffe die Entwicklung von Arthropoden zu hemmen vermögen.

Verwendet man 5-Hydroxy-3,3,6,7-tetramethyl-indan und Methylisocyanat als Ausgangsstoffe, so läßt sich der Reaktionsablauf gemäß Verfahren a) durch folgendes Formelschema wiedergeben:

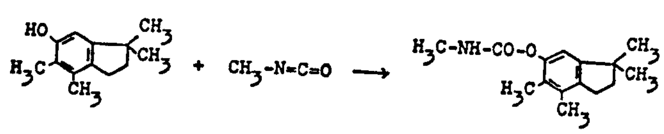

Verwendet man 5-Hydroxy-3,3,6,7-tetramethyl-indan, Phosgen und Methylamin als Ausgangsstoffe, so läßt sich der Reaktionsablauf gemäß Verfahren b) durch das folgende Formelschema wiedergeben:

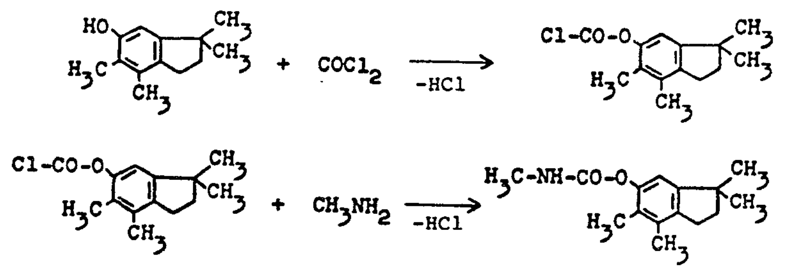


Verwendet man 5-Hydroxy-3,3,6,7-tetramethyl-indan, Phosgen und Methylamin als Ausgangsstoffe, läßt sich der Reaktionsablauf gemäß Verfahren c) durch das folgende Formelschema wiedergeben:

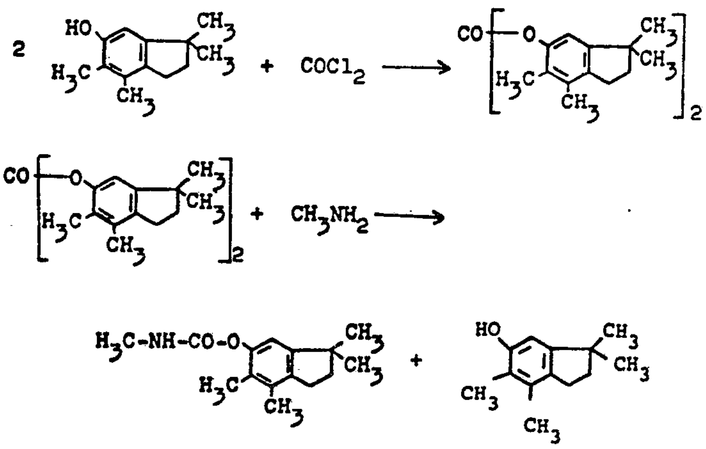

Die als Ausgangsstoffe verwendeten 5-Hydroxy-indane sind durch die oben angegebene Formel (II) eindeutig charakterisiert. In dieser Formel stehen $R^2$ vorzugsweise für Wasserstoff und für niederes Alkyl mit 1 bis 4 C-Atomen und $R^3$ und $R^4$ für Chlor und für niederes Alkyl mit 1 bis 4 C-Atomen. Die Verbindungen lassen sich in Analogie zu bisher bekanntgewordenen Verfahren (vgl. Houben-Weyl, "Methoden der organischen Chemie", 4. Aufl., Band 6/1 c, Seite 987 (1976) und die DT—OS 2 603 835 [Le A 16 930]) durch Umsetzung entsprechender Phenole, wie z.B. 2,3-Dimethylphenol, mit Butadienen, wie z.B. Isopren oder 2,3-Dimethylbutadien-(1,3), in Gegenwart von Phosphorsäure bei Temperaturen von etwa 100 bis 150°C herstellen.

Die weiterhin für die Herstellung der erfindungsgemäßen Verbindungen der Formel (I) benötigten Ausgangsstoffe, Phosgen und Alkylisocyanat für Verfahren a), bzw. Phosgen und Alkylamin für die Verfahrensvarianten b) und c), sind allgemein bekannt. Bevorzugt werden solche Alkylisocyanate bzw. Alkylamine verwendet, die einen Alkylrest mit 1 bis 4 C-Atomen enthalten. Zu nennen sind hier: Methylisocyanat, Äthylisocyanat, Butylisocyanat, sowie die folgenden Amine: Methylamin, Äthylamin, Isopropylamin, Butylamin, Isobutylamin. Demgemäß bedeutet in Formel (I) der Rest $R^1$ vorzugsweise Alkyl mit 1 bis 4 C-Atomen.

Die Reaktion nach Verfahren a) kann in inerten Lösungsmitteln vorgenommen werden. Hierfür eignen sich z.B. Kohlenwasserstoffe, wie Benzin und Benzol, chlorierte Kohlenwasserstoffe, wie Chlorbenzol, aber auch Äther, wie Dioxan, oder Mischungen aus diesen Lösungsmitteln. Die Umsetzung wird durch Zugabe eines tertiären Amins, z.B. Triäthylamin oder Diazabicyclooctan, katalysiert. Die Reaktionstemperaturen können in einem größeren Bereich variiert werden. Im allgemeinen wird man jedoch zwischen 0 und 150°C arbeiten, vorzugsweise zwischen 20 und 110°C.

Arbeitet man nach Verfahrensvariante b), so wird in der ersten Stufe das 5-Hydroxy-indan der Formel (II) zweckmäßigerweise in Gegenwart inerter Lösungsmittel, wie aromatische, gegebenenfalls chlorierte, Kohlenwasserstoffe, so z.B. Benzol, Toluol, Xylol oder Chlorbenzol, mit einem Überschuß an Phosgen in den Chlorkohlensäureester überführt. Die entstehende Salzsäure wird durch Zutropfen einer Base, zweckmäßigerweise Natriumhydroxid, gebunden und so der pH-Wert der Reaktionslösung unter 7 gehalten. Im allgemeinen wird man bei einer Reaktionstemperatur zwischen —20 und +20°C, vorzugsweise zwischen —10 und +10°C, arbeiten. In der zweiten Stufe wird der Chlorkohlensäureester entweder nach Isolierung oder aber direkt in der erhaltenen Reaktionslösung mit der äquivalenten Menge Alkylamin umgesetzt. Dabei arbeitet man ebenfalls zweckmäßigerweise in Gegenwart von inerten Lösungsmitteln wie aromatischen und aliphatischen, gegebenenfalls chlorierten Kohlenwasserstoffen, wie Benzol, Toluol, Chlorbenzol, Benzin, Tetrachlorkohlenstoff, oder Äthern, wie Dioxan. Die Reaktionstemperaturen können wiederum in einem gewissen Bereich variiert werden; man arbeitet im allgemeinen zwischen —20 und +20°C, vorzugsweise zwischen —10 und +10°C.

Arbeitet man schließlich nach Verfahrensvariante c), so wird in der ersten Stufe das 5-Hydroxy-indan der Formel (II) mit der äquivalenten Menge Phosgen zum Bis-(indanyl)-kohlensäureester umgesetzt. Man führt die Reaktion zweckmäßigerweise in inerten Lösungsmitteln, wie aromatischen Kohlenwasserstoffen, z.B. Benzol und Toluol, durch, wobei man die entstehende Salzsäure durch Zusetzen einer Base, vorzugsseise Alkalihydroxid, bindet. Der pH-Wert der Reaktionslösung sollte etwa bei 8 liegen. Die Reaktionstemperatur kann in einem größeren Bereich variieren, sie liegt im allgemeinen zwischen 0 und 100°C, vorzugsweise zwischen +20 und 60°C.

Das in der ersten Stufe gebildete Carbonat wird anschließend mit Alkylamin aufgespalten. Dabei arbeitet man zweckmäßigerweise ohne Lösungsmittel. Die Reaktion kann jedoch auch in Lösungsmitteln durchgeführt werden. Die Reaktionstemperaturen liegen zwischen —30 und +40°C, vorzugsweise zwischen —10 und +20°C.

Die erfindungsgemäßen Wirkstoffe weisen eine starke fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemäßen Wirkstoffe haben ein breites Wirkungsspektrum und können angewandt werden gegen parasitäre Pilze, die oberirdische Pflanzenteile befallen oder die Pflanzen vom Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemäßen Wirkstoffe mit besonders gutem Erfolg zur Bekämpfung von Venturia-Arten, beispielsweise zur Bekämpfung von Apfelschorf (Fusicladium dendriticum) verwendet werden. Dabei ist zu vermerken, daß die Wirkstoffe nicht nur protektiv, sondern auch kurativ wirksam sind.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides,

Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aëdes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die erfindungsgemäßen Wirkstoffe können in die üblichen Formulierungen übergeführt werden, wie Lösungen, Emulsionen, Suspensionen, Pulver, Pasten und Granulate. Diese werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen in wesentlichen in Frage: Aromaten, wie Xylol, Toluol, Benzol oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Dichlordifluormethan oder Trichlorfluormethan; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als Emulgiermittel; nichtionogene und anionische Emulgatoren, wie Polyoxyäthylen-Fettsäure-Ester, Polyoxyäthylen-Fettalkohol-Äther, z.B. Alkylaryl-polyglycol-äther, Alkylsulfonate, Alkylsulfate, Arylsulfonate sowie Eiweißhydrolysate; als Dispergiermittel: z.B. Lignin-Sulfitablaugen und Methylcellulose.

Die erfindungsgemäßen Wirkstoffe können in den Formulierungen in Mischung mit anderen bekannten Wirkstoffen vorliegen, wie Fungiziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Formulierungen enthalten im allgemeinen zwischen 0,1 und 95 Gewichtsprozent Wirkstoff, vorzugsweise zwischen 0,5 und 90%.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Spritzen, Sprühen, Stäuben, Streuen, Trockenbeizen, Feuchtbeizen, Naßbeizen, Schlämmbeizen oder Inkrustieren.

Bei der Verwendung als Blattfungizide können die Wirkstoffkonzentrationen in den Anwendungsformen in einem größeren Bereich variiert werden. Sie liegen im allgemeinen zwischen 0,1 und 0,00001 Gewichtsprozenten, vorzugsweise zwischen 0,05 und 0,0001%.

Bei der Saatgutbehandlung werden im allgemeinen Wirkstoffmengen von 0,001 bis 50 g je Kilogramm Saatgut, vorzugsweise 0,01 bis 10 g benötigt.

Wie schon erwähnt, hemmen die erfindungsgemäßen Verbindungen die Entwicklung von Gliederflüßlern (Arthropoden).

In weiter unten beschriebenen Versuchen wird die Arthropodenentwicklungshemmende Wirkung der erfindungsgemäßen Verbindungen aufgezeigt. Dabei werden während der gesamten angegebenen Entwicklung der Testtiere die morphologischen Veränderungen, wie zur Hälfte verpuppte Tiere, unvollständig geschlüpfte Larven oder Raupen, defekte Flügel, pupale Kutikula bei Imagines etc., als Mißbildungen gewertet. Die Summe der morphologischen Mißbildungen, zusammen mit den während des Häutungsgeschehens oder der Metamorphose abgetöteten Tiere, wird in Prozent der Versuchstiere angegeben.

Bei höheren Konzentrationen besitzen die erfindungsgemäßen Verbindungen auch eine insektizide Wirksamkeit.

Die vielseitigen Anwendungsmöglichkeiten der erfindungsgemäßen Wirkstoffe gehen aus den nachfolgenden Verwendungsbeispielen hervor:

Beispiel A

Fusicladium-Test (Apfel)/Protektiv
Lösungsmittel: 4,7 Gew.-Teile Aceton
Emulgator: 0,3 Gew.-Teile Alkyl-aryl-polyglykoläther
Wasser: 95 Gew.-Teile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie mit einer wäßrigen Konidiensuspension des Apfelschorferregers (Fusicladium dendriticum) inokuliert und 18 Stunden lang in einer Feuchtkammer bei 18 bis 20°C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Die erfindungsgemäßen Verbindungen entsprechend den Herstellungsbeispielen 1 und 2 zeigen dabei eine gute, dem beim Stand der Technik aufgegebenen Vergleichspräparat überlegene Wirkung.

Beispiel B

Fusicladium-Test (Apfel)/Kurativ
Lösungsmittel: 4,7 Gew.-Teile Aceton
Emulgator: 0,3 Gew.-Teile Alkyl-aryl-polyglykoläther
Wasser: 95 Gew.-Teile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Junge Apfelsämlinge, die sich im 4- bis 6-Blattstadium befinden, werden mit einer wäßrigen Konidiensuspension des Apfelschorferregers (Fusicladium dentriticum) inokuliert und 18 Stunden lang in einer Feuchtkammer bei 18 bis 20°C und 100% relativer Luftfeuchtigkeit inkubiert. Die Pflanzen kommen anschließend ins Gewächshaus. Sie trocknen ab.

Nach einer angemesenen Verweilzeit werden die Pflanzen mit der Spritzflüssigkeit, die in der oben angegebenen Weise hergestellt wirde, bis zur Tropfnässe bespritzt. Anschließend kommen die Pflanzen erneut ins Gewächshaus.

15 Tage nach der Inokulation wird der Befall der Apfelsämlinge bestimmt. Die erhaltenen Boniturwerte werden in Prozent Befall umgerechnet. 0% bedeutet keinen Befall, 100% bedeutet, daß die Pflanzen vollständig befallen sind.

Die erfindungsgemäße Verbindung entsprechend dem Herstellungsbeispiel 1 zeigt dabei eine gute, dem beim Stand der Technik angegebenen Vergleichspräparat überlegene Wirkung.

Beispiel C

Entwicklungshemmende Wirkung/Fraßtest
Testtiere: Plutella maculipennis (Raupen im 4. Entwicklungsstadium) 20 Stück
Phaedon cochleariae (Larven im 4. Entwicklungsstadium) 20 Stück
Futterpflanzen: Kohlpflanzen (Brassica oleracea)
Lösungsmittel: 10 Gew.-Teile Dimethylformamid
Emulgator: 1 Gew.-Teil Polyoxyäthylensorbitanmonolaurat

Zur Herstellung einer zweckmäßigen Wirkstoffzubereitung vermischt man 2 Gew.-Teile Wirksto-f mit der angegebenen Menge Lösungsmittel, Emulgator und soviel Wasser, daß eine 1%ige Mischung entsteht, die mit Wasser auf die gewünschte Konzentration verdünnt wird.

Die Testtiere werden mit Blättern der Futterpflanzen, die mit einem gleichmäßigen Spritzbelag der Wirkstoffmischung der gewählten Konzentration versehen sind, so daß die angegebenen Wirkstoffmengen in ppm (parts pro million) auf den Blättern erhalten werden, bis zur Entwicklung der Imago gefüttert.

Zur Kontrolle werden nur mit Lösungsmittel und Emulgator der angegebenen Konzentration versehene Blätter verfüttert.

Die erfindungsgemäße Verbindung entsprechend dem Herstellungsbeispiel 1 zeigt dabei gute Wirksamkeit. Sie übertrifft die Wirkung des bekannten Vergleichspräparates 2,2-Dimethyl-6-methoxybenzopyran.

Beispiel D

Entwicklungshemmende Wirkung
Testtier: Ceratitis capitata (Eier) 20 Stück
Futter: Kunstfutter (Möhrenpulver mit Hefepulver)

Lösungsmittel: 20 Gew.-Teile Aceton
Emulgator: 5 Gew.-Teile Alkylarylpolyglykoläther

Zur Herstellung einer zweckmäßigen Wirkstoffzubereitung vermischt man 4 Gew.-Teile Wirkstoff mit der angegebenen Menge Lösungsmittel, Emulgator und soviel Wasser, daß eine 1%ige Mischung entsteht, die mit Wasser auf die gewünschte Konzentration verdünnt wird.

Die Eier der Testtiere werden auf eine Schale mit Kunstfutter gebracht, in das die gewählte Konzentration der Wirkstoffmischung eingearbeitet ist, so daß die angegebene Wirkstoffmenge in ppm (parts pro million) erreicht wird. Es wird die Entwicklung bis zum Schlupf der Fliegen beobachtet.

Zur Kontrolle wird nur mit Lösungsmittel und Emulgator der angegebenen Konzentration vermischtes Kunstfutter angeboten.

Die erfindungsgemäßen Wirkstoffe entsprechend der Herstellungsbeispiele zeigen dabei gute Wirksamkeit.

*Herstellungsbeispiele*

Beispiel 1

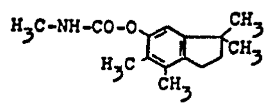

50 g (ca. 0,26 Mol) 5-Hydroxy-3,3,6,7-tetramethyl-indan werden bei Raumtemperatur in 300 ml Ligroin gelöst und mit 20 Tropfen Triäthylamin und 30 g (ca. 0,53 Mol) Methylisocyanat versetzt. Nach dreistündigem Kochen unter Rückfluß läßt man abkühlen. Die Reaktionslösung wird auf Eis gegeben. Die hierbei ausgefallenen Kristalle werden abgesaugt und aus Toluol umkristallisiert. Die Ausbeute beträgt 52,5 g 3,3,6,7-Tetramethylindan-5-yl- N-methyl -carbaminsäureester vom Fp. 148,5—150°C.

Vorprodukt:

Das für obige Synthese benötigte 5-Hydroxy-3,3,6,7-tetramethyl-indan wird wie folgt hergestellt:

Zu einer Lösung von 732 g 2,3-Dimethyl-phenol, 76,5 g 85%iger Phosphorsäure und 6 ml Wasser in 1,44 l o-Dichlor-benzol werden bei 150°C innerhalb von 7 Stunden 449 g Isopren, das mit 1,3 g Phenothiazin stabilisiert ist, zugetropft. Zur Vervollständigung der Reaktion läßt man über Nacht bei 150°C nachrühren. Die Säurephase wird abgetrennt, und die organische Phase wäscht man neutral. Die fraktionierende Destillation ergibt 526 g 5-Hydroxy-3,3,6,7-tetramethyl-indan; $Kp_{15}$: 165—167°C; Fp: 117—118°C.

Beispiel 2

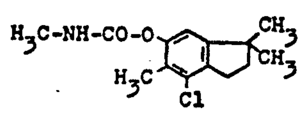

40 g (0,19 Mol) 7-Chlor-5-hydroxy-3,3,6,7-trimethyl-indan werden bei Raumtemperatur in 200 ml Ligroin gelöst und mit 15 Tropfen Triäthylamin und 21,7 g (0,38 Mol) Methylisocyanat versetzt. Nach 24-stündigem Kochen unter Rückfluß läßt man abkühlen. Zur Reaktionslösung werden 400 ml Wasser gegeben. Die hierbei ausgefallenen Kristalle werden abgesaugt, mit Wasser gewaschen, getrocknet und aus Toluol umkristallisiert. Die Ausbeute beträgt 40 g 3,3,6-Trimethyl-7-chlorindan-5-yl-N-methyl -carbaminsäureester vom Fp: 146—146,5°C.

Vorprodukt:

Das für obige Synthese benötigte 7-Chlor-5-hydroxy-3,3,6-trimethyl-indan wird wie folgt hergestellt:

Zu einer Lösung von 832 g 3-Chlor-2-methyl-phenol, 74,4 g 85%iger Phosphorsäure und 5,8 ml Wasser in 1,4 l o-Dichlor-benzol werden bei 110°C innerhalb von 6 Stunden 437 g Isopren, das mit 1,3 g Phenothiazin stabilisiert ist, zugetropft Zur Vervollständigung der Reaktion läßt man über Nacht bei 110°C nachrühren. Die Säurephase wird abgetrennt, und die organische Phase wäscht man neutral. Die fraktionierende Destillation ergibt 296 g 7-Chlor-5-hydroxy-3,3,6-trimethylindan; $Kp_{11}$: 161—164°C; Fp: 109—109,5°C.

In ähnlicher Weise, wie in obligen Beispielen beschrieben, werden die folgenden Verbindungen der allgemeinen Formel

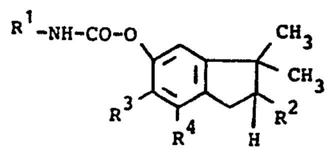

(I)

erhalten:

| Beispiel-Nr. | $R^1$ | $R^2$ | $R^3$ | $R^4$ | Fp (°C) |
|---|---|---|---|---|---|
| 3 | $CH_3$ | $CH_3$ | $CH_3$ | $CH_3$ | 114—114,5 |
| 4 | $C_2H_5$ | H | $CH_3$ | $CH_3$ | 114,5—145 |
| 5 | n-$C_4H_9$ | H | $CH_3$ | $CH_3$ | 110—111 |

**Patentansprüche**

1. Indan-5-yl-N-alkyl-carbaminsäureester der allgemeinen Formel

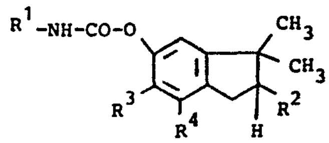

(I)

in welcher
$R^1$ für Alkyl steht,
$R^2$ für Wasserstoff oder Alkyl steht, und
$R^3$ und $R^4$ für Chlor oder Alkyl stehen.

2. Verfahren zur Herstellung von Indan-5-yl-N-alkyl-carbaminsäureestern, dadurch gekennzeichnet, daß man

a) 5-Hydroxy-indane der Formel

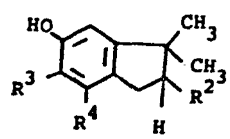

(II)

in welcher
$R^2$, $R^3$ und $R^4$ die oben angegebene Bedeutung besitzen, mit einem Alkylisocyanat umsetzt,
oder

b) 5-Hydroxy-indane der Formel (II) in einer ersten Stufe mit einem Überschuß an Phosgen in den entsprechenden Chlorkohlensäureester überführt und diesen mit Alkylamin umsetzt,
oder

c) 5-Hydroxy-indane der Formel (II) in einer ersten Stufe mit der äquivalenten Menge Phosgen zu dem entsprechenden Bis-(indanyl)-carbonat umsetzt und dieses in einer zweiten Stufe mit Alkylamin aufspaltet.

3. Fungizide und die Entwicklung von Arthropoden hemmende Mittel, gekennzeichnet durch einen Gehalt an mindestens einem Indan-5-yl-N-alkyl-carbaminsäureester gemäß Anspruch 1.

4. Verwendung von Indan-5-yl-N-alkyl-carbaminsäureestern gemäß Anspruch 1 zur Bekämpfung von Pilzen und zur Entwicklungshemmung von Arthropoden.

**Claims**

1. Indan-5-yl N-alkyl-carbamic acid esters of the general formula

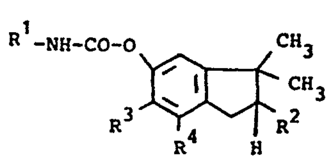

(I)

in which
$R^1$ represents alkyl,
$R^2$ represents hydrogen or alkyl and
$R^3$ and $R^4$ represent chlorine or alkyl.

2. A process for the preparation of indan-5-yl N-alkyl-carbamic acid esters characterised in that

a) 5-hydroxy-indans of the formula

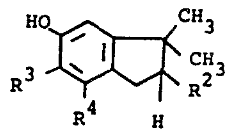

(II)

in which
$R^2$, $R^3$ and $R^4$ have the meaning stated above, are reacted with an alkyl isocyanate, or

b) 5-hydroxy-indans of the formula (II) are converted, in a first step, to the corresponding chlorocarbonic acid ester by means of an excess of phosgene, and this ester is reacted with alkylamine, or

c) 5-hydroxy-indans of the formula (II) are reacted, in a first step, with the equivalent amount of phosgene to give the corresponding bis-indanyl carbonate, and this is split, in a second step, with alkylamine.

3. A fungicidal or arthropod development-inhibiting composition characterised by a content of at least one indan-5-yl N-alkyl-carbamic acid ester according to Claim 1.

4. The use of indan-5-yl N-alkyl-carbamic acid esters according to Claim 1 for combating fungi and for inhibiting the development of arthropods.

**Revendications**

1. Esters indane-5-yliques d'acides N-alkyl-carbamiques répondant à la formule générale:

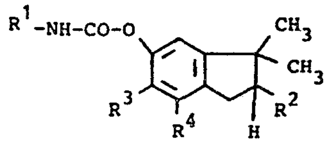

(I)

dans laquelle
$R^1$ représente un groupe alkyle,
$R^2$ représente l'hydrogène ou un groupe alkyle, et
$R^3$ et $R^4$ représentent le chlore ou des groupes alkyle.

2. Procédé de préparation d'esters indane-5-yliques d'acides N-alkyl-carbamiques, ce procédé se caractérisant en ce que:

a) on fait réagir des 5-hydroxy-indanes répondant à la formule:

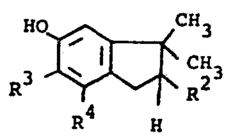

(II)

dans laquelle
$R^2$, $R^3$ et $R^4$ ont les significations indiquées ci-dessus, avec un isocyanate d'alkyle,
ou bien

b) on convertit des 5-hydroxy-indanes répondant à la formule II, dans un premier stade opératoire, en les esters chlorocarboniques correspondants par un excès de phosgène, et on fait réagir ces esters chlorocarboniques avec une alkylamine, ou bien

c) on convertit les 5-hydroxy-indanes de formule II, dans un premier stade opératoire, en le carbonate de bis-(indanyle) correspondant par la quantité équivalente de phosgène et, dans un deuxième stade opératoire, on scinde le carbonate des bis-(indanyle) à l'aide d'une alkylamine.

3. Produits fongicides et inhibiteurs du développement des arthropodes, caractérisés en ce qu'ils contiennent au moins un ester indane-5-ylique d'acide N-alkyl-carbamique selon la revendication 1.

4. Utilisation des esters indane-5-yliques d'acides N-alkyl-carbamiques selon la revendication 1 pour combattre les mycètes et inhiber le développement des arthropodes.